(19) Europäisches Patentamt
European Patent Office
Office européen des brevets

(11) **EP 4 483 822 A1**

(12) **EUROPEAN PATENT APPLICATION**
published in accordance with Art. 153(4) EPC

(43) Date of publication:
01.01.2025 Bulletin 2025/01

(51) International Patent Classification (IPC):
*A61B 17/32* *(2006.01)*

(21) Application number: 23769641.4

(22) Date of filing: 09.03.2023

(86) International application number:
PCT/CN2023/080376

(87) International publication number:
WO 2023/174135 (21.09.2023 Gazette 2023/38)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 15.03.2022 CN 202210249080

(71) Applicant: Hocermed (Beijing) Medical
Technology Co., Ltd.
Beijing 100193 (CN)

(72) Inventor: ZHONG, Baoxuan
Beijing 100193 (CN)

(74) Representative: Murgitroyd & Company
165-169 Scotland Street
Glasgow G5 8PL (GB)

(54) **CONTROL METHOD AND CONTROL DEVICE FOR ULTRASONIC TRANSDUCER, SURGICAL EQUIPMENT, AND STORAGE MEDIUM**

(57) A control method and control device for an ultrasonic transducer, surgical equipment, and a storage medium. The control method comprises: obtaining a target current which flows through a load and enables an ultrasonic transducer to be in a resonant state (S 100); applying a driving signal to the ultrasonic transducer to generate a load current flowing through the load (S200); and adjusting the driving signal to enable the load current to be equal to the target current, such that the ultrasonic transducer is in the resonant state (S300). In the working process of the ultrasonic transducer, the control method can control, in real time, a current flowing through a dynamic branch to be equal to the target current, such that the ultrasonic transducer is always in the resonant state, the impedance is the lowest, and the electromechanical conversion efficiency is the highest.

S100

Obtain a target current I0 which flows through a load and enables an ultrasonic transducer to be in a resonant state I0

S200

Apply a driving signal to the ultrasonic transducer to generate a load current I1 flowing through the load R

S300

Adjust the driving signal to enable the load current I1 to be equal to the target current I0, such that the ultrasonic transducer is in the resonant state

Fig. 4

## Description

### CROSS-REFERENCE TO RELATED APPLICATION(S)

This application is based on and claims the priority to Chinese Patent Application No.202210249080.9 filed on March 15, 2022 and entitled " CONTROL METHOD AND CONTROL DEVICE FOR ULTRASONIC TRANSDUCER, SURGICAL EQUIPMENT AND STORAGE MEDIUM ", the disclosure of the above-mentioned Chinese Patent Application is incorporated herein in its entirety by reference.

### TECHNICAL FIELD

Embodiments of the present disclosure relate to a control method and a control device for an ultrasonic transducer, a surgical equipment and a storage medium.

### BACKGROUND

Ultrasonic scalpel is a common surgical scalpel, which can be widely used in surgical procedures because of its characteristics such as minimum trauma, less smoke and capability of coagulation. The ultrasonic scalpel includes a host, an ultrasonic transducer and an ultrasonic scalpel blade, with the working principle that the host generates a power source with a certain frequency and provides the power source to the ultrasonic transducer, and the ultrasonic transducer generates a mechanical vibration at this frequency and drives the ultrasonic scalpel blade to also generate a mechanical vibration. Since the working frequency of the ultrasonic scalpel is generally 20 kHz~100 kHz, it belongs to high-frequency electrosurgical equipment and acts on human tissues for cutting and coagulation, which will not cause side effects such as tissue drying and burning, and there is no current flowing through the human body either when the blade works.

The core of ultrasonic scalpel lies in how to drive the ultrasonic transducer to convert electric energy into mechanical energy. When the transducer of the ultrasonic scalpel works in a resonant state, it achieves the maximum efficiency of converting electric energy into mechanical energy, and also a longer service life of the ultrasonic scalpel blade. Therefore, it is necessary to match the impedance of the ultrasonic transducer, so that the ultrasonic transducer can always work near the resonance point when the load and external conditions change.

### SUMMARY

Embodiments of the present disclosure provide a control method and a control device for an ultrasonic transducer, a surgical equipment and a storage medium.

According to a first aspect of the present disclosure, a control method for ultrasonic transducer is provided. The ultrasonic transducer is connected with a load and the control method includes: acquiring a target current which flows through the load and enables the ultrasonic transducer to be in a resonant state; applying a driving signal to the ultrasonic transducer to generate a load current flowing through the load; and adjusting the driving signal to enable the load current to be equal to the target current, so that the ultrasonic transducer is in the resonant state.

In at least some embodiments, the driving signal comprises a driving current, and the adjusting the driving signal comprises: acquiring the driving current applied to the ultrasonic transducer; and adjusting the load current by adjusting the driving current to enable the load current to be equal to the target current.

In at least some embodiments, the driving signal comprises a driving current, a driving voltage and a driving frequency, and there is a phase angle between the driving current and the driving voltage, and the adjusting the driving signal comprises: acquiring the driving frequency applied to the ultrasonic transducer; and adjusting the phase angle by adjusting the driving frequency, so as to enable the load current to be equal to the target current.

In at least some embodiments, the adjusting the phase angle by adjusting the driving frequency comprises: determining a target phase angle and a target frequency corresponding to the target phase angle; and adjusting the driving frequency to enable the driving frequency to be equal to the target frequency, so that the phase angle is equal to the target phase angle.

In at least some embodiments, the ultrasonic transducer is equivalent to a static capacitor, the static capacitor is connected in parallel with the load, and the determining a target phase angle comprises: acquiring the driving voltage applied to the ultrasonic transducer and a capacitive reactance of the ultrasonic transducer; determining a current flowing through the static capacitor, according to the driving voltage and the capacitive reactance; and determining the target phase angle, according to the current flowing through the static capacitor and the target current.

In at least some embodiments, determining the target phase angle according to the current flowing through the static capacitor and the target current by the following equation:

$$\theta 0 = \arctan \frac{I2}{I0}$$

where $\theta 0$ is the target phase angle, $I2$ is the current flowing through the static capacitor, and $I0$ is the target current.

In at least some embodiments, the determining a target frequency corresponding to the target phase angle comprises: performing frequency scanning within a preset frequency range; and acquiring a frequency that enables the ultrasonic transducer to be in the resonant state as the target frequency during the frequency scanning.

In at least some embodiments, when the ultrasonic transducer is at a frequency in the resonant state, the ultrasonic transducer has a minimum impedance.

In at least some embodiments, the preset frequency range is greater than or equal to 53.5 kHz and less than or equal to 57.5 kHz; the target frequency is greater than or equal to 53.5 kHz and less than or equal to 57.5 kHz; and the target phase angle is greater than or equal to 0° and less than or equal to 90°.

In at least some embodiments, the driving signal comprises a driving current, a driving voltage and a driving frequency, and there is a phase angle between the driving current and the driving voltage, the control method further comprises: acquiring the driving current and the driving frequency applied to the ultrasonic transducer; and adjusting the driving current and the driving frequency to adjust the phase angle, so that the load current is equal to the target current.

In at least some embodiments, the load current, the driving current and the phase angle satisfy the following equation:

$$I1 = I \times \cos\theta,$$

where I1 is the load current, I is the driving current, and $\theta$ is the phase angle.

In at least some embodiments, the ultrasonic transducer is connected with an ultrasonic scalpel blade, and the ultrasonic scalpel blade is configured to cut a target tissue; during cutting the target tissue by the ultrasonic scalpel blade, repeatedly executing the step of acquiring a target current which flows through the load and enables the ultrasonic transducer to be in a resonant state, the step of applying a driving signal to the ultrasonic transducer to generate a load current flowing through the load and the step of adjusting the driving signal to enable the load current to be equal to the target current so that the ultrasonic transducer is in the resonant state, until the target tissue is cut off.

According to a second aspect of the present disclosure, a control device for ultrasonic transducer, which is connected with a load, and the control device comprises an acquisition unit configured to acquire a target current which flows through the load and enables the ultrasonic transducer to be in a resonant state; a driving unit configured to apply a driving signal to the ultrasonic transducer to generate a load current flowing through the load; An adjusting unit configured to adjust the driving signal so that the load current is equal to the target current, thereby placing the ultrasonic transducer in the resonant state.

According to a third aspect of that present disclosure, there is provided a surgical equipment comprise a host and an ultrasonic transducer connected with the host, wherein the host comprises the control device.

According to a fourth aspect of that present disclosure, there is provided a surgical equipment comprise a processor; A memory including one or more computer program modules; Wherein the one or more computer program modules are stored in the memory and configured to be executed by the processor, and the one or more computer program modules include instructions for executing the aforementioned control method.

According to a fifth aspect of that present disclosure, there is provided a storage medium for non-temporarily store computer-readable instructions which, when executed by a computer, can realize the aforementioned control method.

BRIEF DESCRIPTION OF DRAWINGS

In order to explain the technical solution of the embodiments of the present disclosure more clearly, the accompanying drawings of the embodiments will be briefly introduced below. Obviously, the accompanying drawings in the following description only relate to some embodiments of the present disclosure, and are not intended to limit the present disclosure.

Fig. 1 is an equivalent circuit of a surgical equipment with an ultrasonic transducer of the present disclosure;

Fig. 2 is a block diagram illustrating connections of various components of an ultrasonic scalpel according to an embodiment of the present disclosure;

Fig. 3 is an equivalent circuit of a surgical equipment with an ultrasonic transducer according to an embodiment of the present disclosure;

Fig. 4 is a flowchart of a control method for an ultrasonic transducer provided by an embodiment of the present disclosure;

Fig. 5 is a flowchart illustrating a step of adjusting a driving signal which is executed in a control method according to an embodiment of the present disclosure;

Fig. 6 is a flowchart illustrating a step of adjusting a driving frequency which is executed in a control method according to an embodiment of the present disclosure;

Fig. 7 is a schematic diagram illustrating a relationship between a driving current and a dynamic branch current according to an embodiment of the present disclosure;

Fig. 8 is a flowchart illustrating a step of adjusting a driving signal which is executed in a control method according to another embodiment of the present disclosure;

Fig. 9 is a flowchart illustrating a step of determining a target phase angle which is executed in a control method according to an embodiment of the present disclosure;

Fig. 10 is a flowchart illustrating a step of determining a target frequency which is executed in a control method according to an embodiment of the present disclosure; and

Fig. 11 is a block diagram of a control device for an ultrasonic transducer according to an embodiment of the present disclosure.

DETAILED DESCRIPTION

In order to make objects, technical details and advantages of embodiments of the present disclosure clear, the technical solutions of the embodiments will be described in a clearly and fully understandable way in connection with the related drawings. It is apparent that the described embodiments are just a part but not all of the embodiments of the present disclosure. Based on the described embodiments herein, those skilled in the art can obtain, without any inventive work, other embodiment(s) which should be within the scope of the present disclosure.

Unless otherwise defined, all the technical and scientific terms used herein have the same meanings as commonly understood by one of ordinary skill in the art to which the present disclosure belongs. The terms "first," "second," etc., which are used in the description and claims of the present disclosure, are not intended to indicate any sequence, amount or importance, but distinguish various components. The terms "comprises," "comprising," "includes," "including," etc., are intended to specify that the elements or the objects stated before these terms encompass the elements or the objects listed after these terms as well as equivalents thereof, but do not exclude other elements or objects. The phrases "connect", "connected", etc., are not intended to define a physical connection or a mechanical connection, but may comprise an electrical connection which is direct or indirect. The terms "on," "under," "right," "left" and the like are only used to indicate relative position relationship, and in a case that the position of an object is described as being changed, the relative position relationship may be changed accordingly.

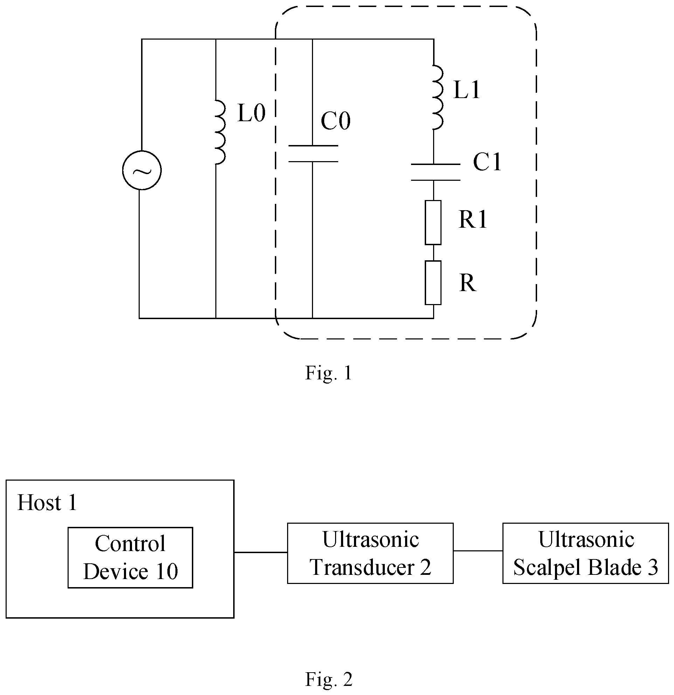

Fig. 1 is an equivalent circuit of a surgical equipment with an ultrasonic transducer of the present disclosure. The surgical equipment is, for example, an instrument for surgical procedure, such as an ultrasonic scalpel. When the ultrasonic scalpel blade cuts the target tissue, the ultrasonic transducer and the ultrasonic scalpel blade, as a whole, are in a mechanical resonant state, which can be equivalent to the circuit of Fig. 1 in a resonant state.

The equivalent circuit of the ultrasonic transducer is shown in the dashed box of Fig. 1, in which L1 is a dynamic inductor, C1 is a dynamic capacitor and R1 is a dynamic resistor; the dynamic inductor L1 is caused by a mass of the transducer, and R1 is caused by a mechanical loss of the transducer. In the present disclosure, a branch including the dynamic inductor L1, the dynamic capacitor C1 and the dynamic resistor R1 is referred to as a dynamic branch. C0 is a static capacitor, which is the capacitance between the two electrode plates of the clamped piezoelectric vibrator and is connected in parallel with the dynamic branch. R is a load resistor, which is located in the dynamic branch and is connected in series with each of the dynamic inductor L1, the dynamic capacitor C1 and the dynamic resistor R1; when there is no load, R is zero.

During the working of the ultrasonic scalpel, the host of the ultrasonic scalpel generates an electrical signal with a certain frequency, which frequency enables the dynamic branch to reach the minimum impedance so as to achieve the maximum efficiency. According to the principle of the series resonant circuit, the frequency should satisfy: $f = 1/2\pi\sqrt{L1 * C1}$ . At this time, the ultrasonic transducer works in a series resonant state, and the dynamic branch is equivalent to the case where only the dynamic resistor R1 exists, with the minimum impedance and the maximum efficiency. However, due to the existence of the static capacitor C0, the ultrasonic transducer exhibits a capacitive property, which limits the working efficiency of the ultrasonic transducer even if it works at the series resonant frequency.

As shown in Fig. 1, in order to improve the working efficiency of the ultrasonic transducer, an inductor L0 is usually connected in parallel or in series with the dynamic branch (only a parallel connection is shown in Fig. 1), so that the inductor L0 and the static capacitor C0 also work in a resonant state at the current working frequency.

The inventor(s) found that the load R will change in real time during the working of the ultrasonic scalpel because the target tissue being cut may be different. For example, when cutting some hard tissues, the load impedance will increase, so that the resonant frequency of the transducer in the resonant state is not fixed. Moreover, the static capacitor C0 also varies for different ultrasonic transducers, which also affects the resonant frequency. Since the ultrasonic transducer is a lossy component, its static capacitor C0 will change after a period of using time, with considerable discreteness. For example, the longer the service life is, the higher the static capacitor C0 will be, resulting in a dynamic change of the resonant frequency of the ultrasonic transducer during the cutting of the target tissue.

In view of the above, an embodiment of the present disclosure provides a control method for an ultrasonic transducer. The ultrasonic transducer is connected with a load, and the control method includes: acquiring a target current which flows through the load and enables the ultrasonic transducer to be in a resonant state; applying a driving signal to the ultrasonic transducer to generate a load current flowing through the load; and adjusting the driving signal to enable the load current to be equal to the target current, so that the ultrasonic transducer is in the resonant state.

In the control method for an ultrasonic transducer in the above embodiment of the present disclosure, the load current flowing through the load is made equal to the target current by adjusting the driving signal, so that the ultrasonic transducer is always in a resonant state.

In the embodiment of the present disclosure, the current of the dynamic branch that flows through the load resistor determines the amplitude of the ultrasonic scalpel blade. In order to achieve accurate tissue cutting and good hemostasis effect, it is necessary to control the amplitude. In order to control the amplitude, it is necessary to control the current flowing through the dynamic branch in real time. Through the control method disclosed by the present disclosure, the current flowing through the dynamic branch can be controlled, in real time, to be equal to the target current during the working of the ultrasonic scalpel, so that the ultrasonic transducer is always in a resonant state, with the minimum impedance and the maximum efficiency of electromechanical conversion.

The present disclosure will be explained through specific embodiments. In order to keep the following description of the embodiments of the present disclosure clear and concise, detailed explanations of known functions and known components may be omitted. When any component of an embodiment of the present disclosure appears in more than one drawing, the component may be represented by the same reference numeral in each drawing.

Fig. 2 is a block diagram illustrating connections of various components of an ultrasonic scalpel according to an embodiment of the present disclosure. The embodiment of the present disclosure provides a surgical equipment, for example, an instrument for surgical procedure. The instrument for surgical procedure is, for example, an ultrasonic scalpel.

As shown in Fig. 2, the ultrasonic scalpel of the embodiment of the present disclosure includes a host 1, an ultrasonic transducer 2 and an ultrasonic scalpel blade 3. For example, the host 1 is connected with the ultrasonic transducer 2, and the ultrasonic transducer 2 is connected with the ultrasonic scalpel blade 3. The host 1 includes, for example, a control device 10 that provides a driving signal to the ultrasonic transducer 2. When excited by the driving signal, the ultrasonic transducer 2 generates a mechanical vibration and transmits the mechanical vibration to the ultrasonic scalpel blade 3. The ultrasonic scalpel blade 3 cuts a target tissue or stops bleeding through high-frequency vibration.

For example, in an example, the ultrasonic scalpel blade 3 includes a rod-shaped assembly including a proximal end and a distal end along its extending direction. The ultrasonic transducer 2 includes a housing having a hand-held portion convenient for hand-holding, and the housing is connected to the proximal end of the rod-shaped assembly. The distal end of the rod-shaped assembly is provided with an end effector which can be switched between an open state and a closed state to perform the action of cutting the target tissue. For the specific structure of the ultrasonic scalpel blade 3 and its connection with the ultrasonic transducer 2, reference can be made to the existing designs, and will not be described in detail here.

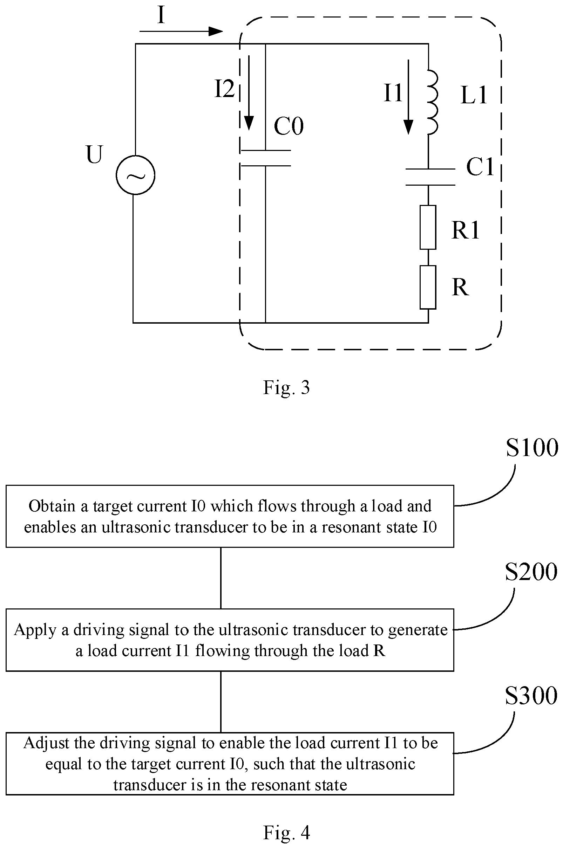

Fig. 3 is an equivalent circuit of a surgical equipment with an ultrasonic transducer according to an embodiment of the present disclosure. Fig. 4 is a flowchart of a control method for an ultrasonic transducer provided by an embodiment of the present disclosure.

As shown in Fig. 3, in the surgical equipment of the embodiment of the present disclosure, the equivalent circuit of the ultrasonic transducer 2 is shown by a dotted line, including a dynamic inductor $L1$, a dynamic capacitor $C1$, a dynamic resistor $R1$ and a load resistor $R$ which are connected in series; the equivalent circuit further includes a static capacitor $C0$ which is connected in parallel with a dynamic branch consisting of the dynamic inductor $L1$, the dynamic capacitor $C1$, the dynamic resistor $R1$ and the load resistor $R$. In the ultrasonic scalpel of the embodiment of the present disclosure, there is no need to set the matching inductor $L0$ as shown in Fig. 1, which simplifies the design of the matching circuit.

For example, the driving signal includes a driving current $I$ and a driving voltage $U$. Based on the equivalent circuit of Fig. 3, the driving current $I$ is divided into two branch currents $I1$ and $I2$, where the branch current $I1$ is the load current flowing through the load resistor $R$ and the branch current $I2$ is the current flowing through the static capacitor $C0$. In the process of cutting the target tissue by the ultrasonic scalpel, the load resistor $R$ changes due to the different hardness and softness of the target tissue. The inventor(s) found that it is necessary to ensure the amplitude of the ultrasonic scalpel blade 3 to be within a predetermined range in order to achieve good cutting or hemostasis effect, and the amplitude of the ultrasonic scalpel blade 3 is determined by the current $I1$ flowing through the load resistor $R$.

Therefore, as shown in Figs. 3 and 4, the control method for an ultrasonic transducer provided by the embodiment of the present disclosure includes:

S100, acquiring a target current $I0$ which flows through the load and enables the ultrasonic transducer to be in a resonant state;

S200: applying a driving signal to the ultrasonic transducer to generate a load current I1 flowing through the load R;

S300: adjusting the driving signal to enable the load current I1 to be equal to the target current I0, so that the ultrasonic transducer is in the resonant state.

As mentioned earlier, the static capacitor C0 has considerable discreteness in different ultrasonic transducers; and due to the limitation of winding process for inductors, there is also great discreteness; this results in different resonant frequencies for various ultrasonic transducers. At the same time, the change of the load impedance will also cause a fluctuation of the resonant frequency during the cutting of the target tissue. In the above control method, the load current I1 flowing through the load can be made equal to the target current I0 by adjusting the driving signal, so that the ultrasonic transducer 2 is always in a resonant state in the process of cutting the target tissue.

For example, the target current I0 is the current that enables the ultrasonic transducer 2 to be in a resonant state, which is detected for the ultrasonic transducer 2 at the factory. In an example, the target current I0 is in the range from 125 mA to 400 mA.

In the embodiment of the present disclosure, "A equals B" includes the case that A is within a preset range of B. For example, the fact that the load current I1 is equal to the target current I0 includes the case where the load current I1 is within a preset range of the target current I0. For example, when the load current I1 fluctuates within the preset range (for example, ±10%) of the target current I0, it belongs to the category of "the load current I1 is equal to the target current I0". That is, the load current I1 may be greater than or equal to the target current I0 multiplied by 90% and less than or equal to the target current I0 multiplied by 110%. Further, the load current I1 can be greater than or equal to the target current I0 multiplied by 95% and less than or equal to the target current I0 multiplied by 105%, thereby further improving the control accuracy of the load current I1.

Similarly, the expression "the driving frequency f is equal to the target frequency f0" mentioned below can be understood as: when the driving frequency f fluctuates within a preset range (for example, ±10%) of the target frequency f0, it belongs to the category of "the driving frequency f is equal to the target frequency f0". The expression "the phase angle θ is equal to the target phase angle θ0" mentioned below can be understood as: when the phase angle θ fluctuates within a preset range (for example, ±10%) of the target phase angle θ0, it belongs to the category of "the phase angle θ is equal to the target phase angle θ0".

Generally, there is a phase difference between the driving current I and the driving voltage U, and the phase difference is also referred to as the phase angle θ. In practical working, the driving signal is, for example, a sine wave signal, which can be converted into a phase square wave signal of voltage and current; further, the phase square wave signal can be converted into a phase difference square wave signal. Thus, the phase difference value can be obtained.

Since the sine wave signal can reflect the frequency of a signal, the driving signal also includes a driving frequency f. The driving frequency f is related to the phase angle θ. When the ultrasonic transducer 2 is at the resonant frequency, the driving frequency f is equal to the target frequency f0, that is, the resonant frequency. At this time, the corresponding phase angle θ is referred to as the target phase angle θ0.

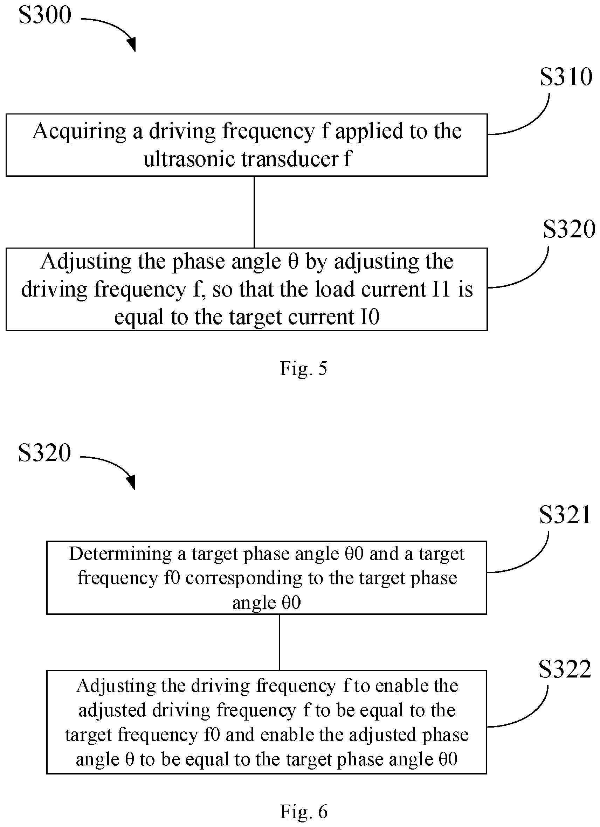

Fig. 5 is a flowchart illustrating the step of adjusting the driving signal, which is executed in the control method of the embodiment of the present disclosure. Fig. 6 is a flowchart illustrating the step of adjusting the driving frequency, which is executed in the control method of the embodiment of the present disclosure.

As shown in Fig. 5, when adjusting the driving signal in step 300, the control method includes:

S310, acquiring a driving frequency f applied to the ultrasonic transducer; and

S320, adjusting the phase angle θ by adjusting the driving frequency f, so that the load current I1 is equal to the target current I0.

Further, as shown in Fig. 6, for example, when adjusting the driving frequency in step 200, the control method includes:

S321: determining a target phase angle θ0 and a target frequency f0 corresponding to the target phase angle θ0;

S322: adjusting the driving frequency f to enable the adjusted driving frequency f to be equal to the target frequency f0 and enable the adjusted phase angle θ to be equal to the target phase angle θ0.

The inventor(s) found that during the working of the ultrasonic transducer 2, the driving frequency f determines the phase angle θ, which further determines the distribution of the driving current I in the two branches of Fig. 3, that is, the proportional relationship between the load current I1 and the current I2 flowing through the static capacitor C0. Therefore, by adjusting the phase angle θ through controlling the driving frequency f, the proportional relationship between the load current I1 and the current I2 can be adjusted.

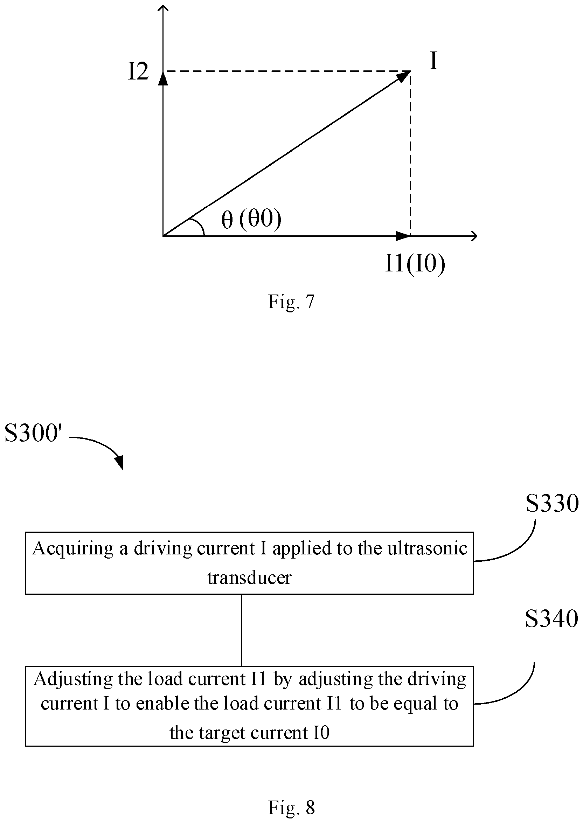

Fig. 7 is a schematic diagram illustrating the relationship between the driving current and the dynamic branch current according to the embodiment of the present disclosure. As shown in Figs. 3 and 7, the driving current I, the load current I1 and the current I2 flowing through the static capacitor C0 satisfy the following trigonometric function relationship:

$$\sin\theta = I2/I, \cos\theta = I1/I.$$

When the phase angle θ is adjusted to θ0, the proportional relationship between I2 and I1 can be determined. At this time, by further adjusting the magnitude of the driving current I, the magnitude of the load current I1 can be adjusted, so that the load current I1 is equal to the target current I0.

Fig. 8 is a flowchart illustrating the step of adjusting the driving signal, which is executed in the control method according to another embodiment of the present disclosure. As shown in Fig. 8, when adjusting the driving signal in step 300', the control method includes:

> S330, acquiring a driving current I applied to the ultrasonic transducer; and
>
> S340, adjusting the load current I1 by adjusting the driving current I to enable the load current I1 to be equal to the target current I0.

In the embodiment of the present disclosure, the step S322 of adjusting the driving frequency f and the step S340 of adjusting the driving current I can be executed simultaneously or one after the other, for example, the driving frequency f is adjusted firstly and then the driving current I is adjusted, which is not limited in the embodiment of the present disclosure.

In the embodiments of the present disclosure, the target phase angle θ0 and the target driving frequency f0 can be obtained in various ways, one of which is provided below. It can be understood that all the methods of obtaining the target phase angle θ0 and the target driving frequency f0 in the process of cutting the target tissue are fallen into the scope of the embodiments of the present disclosure.

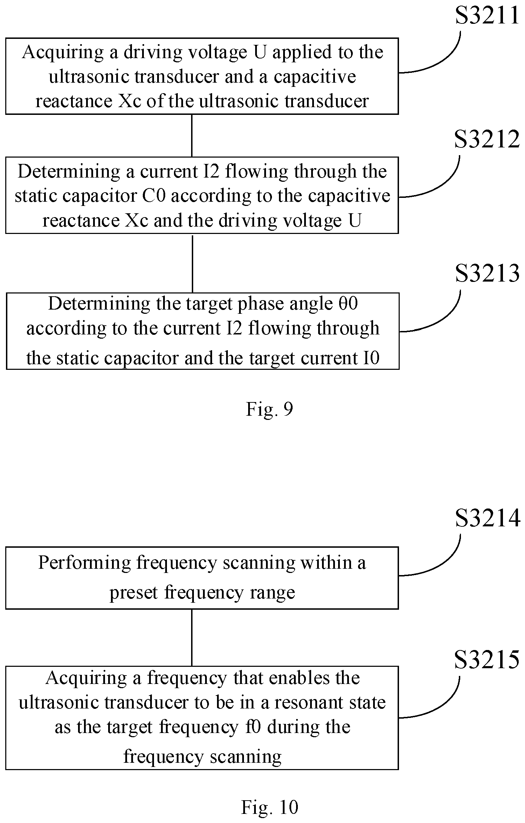

Fig. 9 is a flowchart illustrating the step of determining the target phase angle, which is executed in the control method of the embodiment of the present disclosure. As shown in Fig. 9, when determining the target phase angle θ0, the control method includes:

> S3211, acquiring a driving voltage U applied to the ultrasonic transducer and a capacitive reactance Xc of the ultrasonic transducer;
> S3212, determining a current I2 flowing through the static capacitor C0, according to the capacitive reactance Xc and the driving voltage U;
> S3213, determining the target phase angle θ0, according to the current I2 flowing through the static capacitor and the target current I0.

For example, in step S3211, the capacitive reactance Xc is calculated according to the following formula (1):

$$Xc = \frac{1}{jwC} \quad (1)$$

where w is an angular frequency, C is a capacitance value of the static capacitor C0, and j is the sign of imaginary number in a complex number. For example, w=2πf, and f is the frequency.

For example, in step S3212, based on the calculated capacitive reactance Xc and the obtained driving voltage U, the current I2 flowing through the static capacitor C0 is calculated according to the following formula (2):

$$I2 = \frac{U}{Xc} \quad (2)$$

For example, in step S3213, with reference to the trigonometric function relationship shown in Fig. 7, based on the calculated current I2 and the target current I0, the target phase angle θ0 is calculated according to the following formula (3):

$$\theta0 = \arctan\frac{I2}{I0} \quad (3)$$

At this point, the target phase angle θ0 is obtained.

Fig. 10 is a flowchart illustrating the step of determining the target frequency, which is executed in the control method of the embodiment of the present disclosure. As shown in Fig. 10, when determining the target frequency f0, the control method includes:

> S3214: performing frequency scanning within a preset frequency range;
>
> S3215: acquiring a frequency that enables the ultrasonic transducer to be in a resonant state as the target frequency f0 during the frequency scanning.

In the embodiment of the present disclosure, when the ultrasonic transducer is in a resonant state, that is, when the driving frequency f is equal to the resonant frequency f0, the ultrasonic transducer has the minimum impedance, which not only facilitates the ultrasonic transducer to achieve the maximum working efficiency, but also prolongs the service life of the ultrasonic transducer.

For example, during the frequency scanning, the impedance of the ultrasonic transducer is obtained, and the frequency corresponding to the point with the minimum impedance is taken as the target frequency f0. In an example, the value of the minimum impedance is greater than or equal to 200 ohms.

In the embodiment of the present disclosure, the preset frequency range is greater than or equal to 53.5 kHz and less than or equal to 57.5 kHz; the target frequency f0 is greater than or equal to 53.5 kHz and less than or equal to 57.5 kHz; the target phase angle θ0 is greater than or equal to 0° and less than or equal to 90°.

In the embodiment of the present disclosure, the control method provided by the above embodiment of the present disclosure is repeated in the process of cutting the target tissue by the ultrasonic scalpel blade 3, that is, the step of acquiring the target current I0 which flows through the load and enables the ultrasonic transducer to be in a resonant state, the step of applying a driving signal to the ultrasonic transducer to generate the load current I1 flowing through the load R, and the step of adjusting the driving signal to enable the load current I1 to be equal to the target current I0 are repeatedly executed, so that the ultrasonic transducer is always kept in a resonant state during the working process until the target tissue is completely cut off.

In the control method provided by the embodiment of the present disclosure, the load current can be monitored and adjusted in real time in the process of cutting the target tissue, so that the load current is equal to the target current I0, thereby keeping the ultrasonic transducer 2 in a resonant state all the time. At the same time, compared with the equivalent circuit shown in Fig. 1, since the control method of the present application does not need to consider the size of the inductor L0, it not only lowers the cost of circuit components, but also reduces the calculation error caused by the inductor L0 as compared with the existing control method, thereby improving the control accuracy of the load current.

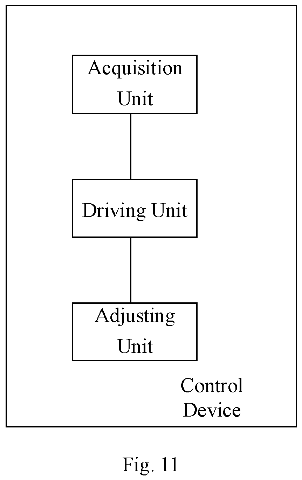

Fig. 11 is a block diagram of a control device for an ultrasonic transducer according to an embodiment of the present disclosure. As shown in Fig. 11, an embodiment of the present disclosure further provides a control device for an ultrasonic transducer. The control device, may be, for example, the control device 10 in Fig. 2, and includes:

> an acquisition unit, configured to acquire a target current I0 which flows through a load R and enables the ultrasonic transducer 2 to be in a resonant state;

> a driving unit, configured to apply a driving signal to the ultrasonic transducer 2 to generate a load current I1 flowing through the load R; and

> an adjusting unit, configured to adjust the driving signal to enable the load current I1 to be equal to the target current I0, so that the ultrasonic transducer 2 is in the resonant state.

In the control device for an ultrasonic transducer in the above embodiment of the present disclosure, the driving signal is adjusted by the adjusting unit to enable the load current flowing through the load to be equal to the target current, so that the ultrasonic transducer is always in a resonant state.

For example, the acquisition unit is further configured to acquire the driving current I applied to the ultrasonic transducer 2, and the adjusting unit is further configured to adjust the load current I1 by adjusting the driving current I so that the load current I1 is equal to the target current I0.

For example, the acquisition unit is further configured to acquire the driving frequency f applied to the ultrasonic transducer, and the adjusting unit is further configured to adjust the phase angle θ by adjusting the driving frequency f, so that the load current I1 is equal to the target current I0.

Further, for example, the adjusting unit is further configured to determine a target phase angle θ0 and a target frequency f0 corresponding to the target phase angle θ0, and adjust the driving frequency f so that the adjusted driving frequency f is equal to the target frequency f0 and the adjusted phase angle θ is equal to the target phase angle θ0. Specific methods for determining the target phase angle θ0 and the target frequency f0 can be found in the previous description, and will not be repeated here.

In the above control device of the embodiment of the present disclosure, the adjusting unit is configured to adjust the driving current I and the driving frequency f, so that the load current can be quickly made equal to the target current, and thus the ultrasonic transducer is always in a resonant state. Moreover, since an inductor L0 connected in series or in parallel, as shown in Fig. 1, is not provided in the control device of the present application, not only the cost of circuit components is lowered but also the calculation error caused by the inductor L0 is reduced compared with the existing control device, thereby improving the control accuracy of the load current.

In some embodiments, when adjusting the phase angle by adjusting the driving frequency, the adjusting unit is further configured to determine a target phase angle and a target frequency corresponding to the target phase angle; and adjust the driving frequency to enable the driving frequency to be equal to the target frequency, so that the phase angle is equal to the target phase angle.

Further, for example, the ultrasonic transducer is equivalent to a static capacitor, and the static capacitor is connected in parallel with the load. In this case, when determining the target phase angle, the adjusting unit is further configured to acquire a driving voltage applied to the ultrasonic transducer and a capacitive reactance of the ultrasonic transducer; determine the current flowing through the static capacitor according to the driving voltage and capacitive reactance; and determine the target phase angle according to the current flowing through the static capacitor and the target current.

Further, for example, according to the current flowing through the static capacitor and the target current, the target phase angle is determined as:

$$\theta0 = \arctan \frac{I2}{I0},$$

where θ0 is the target phase angle, I2 is the current flowing through the static capacitor, and I0 is the target current.

In some embodiments, when determining the target frequency corresponding to the target phase angle, the adjusting unit is further configured to perform frequency scanning within a preset frequency range; and acquire, during the frequency scanning, a frequency that enables the ultrasonic transducer to be in a resonant state as the target frequency.

Further, for example, when the ultrasonic transducer is at a frequency in a resonant state, the ultrasonic transducer has the minimum impedance.

Further, for example, the preset frequency range is greater than or equal to 53.5 kHz and less than or equal to 57.5 kHz; the target frequency is greater than or equal to 53.5 kHz and less than or equal to 57.5 kHz; the target phase angle is greater than or equal to 0° and less than or equal to 90°.

In some embodiments, the acquisition unit is configured to acquire the driving current and the driving frequency. At this time, the adjusting unit is further configured to adjust the driving current and the driving frequency to adjust the phase angle so that the load current is equal to the target current.

In some embodiments, the load current, the driving current and the phase angle satisfy the following relationship:

$$I1 = I \times \cos\theta,$$

where I1 is the load current, I is the driving current, and θ is the phase angle.

In some embodiments, the control device is configured to repeatedly perform the step of acquiring a target current which flows through the load and enables the ultrasonic transducer to be in a resonant state, the step of applying a driving signal to the ultrasonic transducer to generate a load current flowing through the load, and the step of adjusting the driving signal to enable the load current to be equal to the target current so that the ultrasonic transducer is in the resonant state.

The embodiment of the present disclosure further provides a surgical equipment, which includes a processor and a memory, wherein the memory includes one or more computer program modules. The one or more computer program modules are stored in the memory and configured to be executed by the processor, and the one or more computer program modules include instructions for executing the control method provided in any of the previous embodiments.

Embodiments of the present disclosure further provide a storage medium for non-temporarily storing computer-readable instructions, which, when executed by a computer, can realize the control method provided in any of the previous embodiments.

In the present disclosure, there are the following points to be noted.

(1) The drawings of the embodiment of the present disclosure only relate to the structure related to the embodiments of the present disclosure, and other structures can refer to the general designs.

(2) In case of no conflict, the embodiments of the present disclosure and the features in the embodiments can be combined with each other to obtain new embodiment(s).

The above are only the specific embodiments of the present disclosure, but the scope of protection of the present disclosure is not limited to this. Any skilled familiar with the art can easily think of changes or substitutions within the technical scope disclosed in the present disclosure, and they should be included in the scope of protection of the present disclosure. Therefore, the scope of protection of the present disclosure should be based on the scope of protection of the claims.

## Claims

1. A control method for an ultrasonic transducer, **characterized in that**, the ultrasonic transducer being connected with a load and the control method comprising:

    acquiring a target current which flows through the load and enables the ultrasonic transducer to be in a resonant state;
    applying a driving signal to the ultrasonic transducer to generate a load current flowing through the load; and
    adjusting the driving signal to enable the load current to be equal to the target current, so that the ultrasonic transducer is in the resonant state.

2. The control method according to claim 1, **characterized in that**, the driving signal comprises a driving current, and the adjusting the driving signal comprises:

    acquiring the driving current applied to the ultrasonic transducer; and
    adjusting the load current by adjusting the driving current to enable the load current to be equal to the target current.

3. The control method according to claim 1, **characterized in that**,

the driving signal comprises a driving current, a driving voltage and a driving frequency, and there is a phase angle between the driving current and the driving voltage, and the adjusting the driving signal comprises:

acquiring the driving frequency applied to the ultrasonic transducer; and adjusting the phase angle by adjusting the driving frequency, so as to enable the load current to be equal to the target current.

4. The control method according to claim 3, **characterized in that**, the adjusting the phase angle by adjusting the driving frequency comprises:

determining a target phase angle and a target frequency corresponding to the target phase angle; and adjusting the driving frequency to enable the driving frequency to be equal to the target frequency, so that the phase angle is equal to the target phase angle.

5. The control method according to claim 4, **characterized in that**, the ultrasonic transducer is equivalent to a static capacitor, the static capacitor is connected in parallel with the load, and the determining a target phase angle comprises:

acquiring the driving voltage applied to the ultrasonic transducer and a capacitive reactance of the ultrasonic transducer; determining a current flowing through the static capacitor, according to the driving voltage and the capacitive reactance; and determining the target phase angle, according to the current flowing through the static capacitor and the target current.

6. The control method according to claim 5, **characterized in that**, determining the target phase angle according to the current flowing through the static capacitor and the target current by the following equation:

$$\theta0 = \arctan\frac{I2}{I0},$$

where $\theta0$ is the target phase angle, $I2$ is the current flowing through the static capacitor, and $I0$ is the target current.

7. The control method according to claim 4, **characterized in that**, the determining a target frequency corresponding to the target phase angle comprises:

performing frequency scanning within a preset frequency range; and acquiring a frequency that enables the ultrasonic transducer to be in the resonant state as the target frequency during the frequency scanning.

8. The control method according to claim 7, **characterized in that**, when the ultrasonic transducer is at a frequency in the resonant state, the ultrasonic transducer has a minimum impedance.

9. The control method according to claim 7, **characterized in that**,

the preset frequency range is greater than or equal to 53.5 kHz and less than or equal to 57.5 kHz; the target frequency is greater than or equal to 53.5 kHz and less than or equal to 57.5 kHz; and the target phase angle is greater than or equal to 0° and less than or equal to 90°.

10. The control method according to claim 1, **characterized in that**,

the driving signal comprises a driving current, a driving voltage and a driving frequency, and there is a phase angle between the driving current and the driving voltage, the control method further comprises:

acquiring the driving current and the driving frequency applied to the ultrasonic transducer; and adjusting the driving current and the driving frequency to adjust the phase angle, so that the load current is equal to the target current.

11. The control method according to claim 10, **characterized in that**, the load current, the driving current and the phase angle satisfy the following equation:

$$I1 = I \times \cos\theta,$$

where $I1$ is the load current, $I$ is the driving current, and $\theta$ is the phase angle.

12. The control method according to any one of claims 1-11, **characterized in that**:

the ultrasonic transducer is connected with an ultrasonic scalpel blade, and the ultrasonic scalpel blade is configured to cut a target tissue; during cutting the target tissue by the ultrasonic scalpel blade, repeatedly executing the step of acquiring a target current which flows through the load and enables the ultrasonic transducer to be in a resonant state, the step of applying a driving signal to the ultrasonic transducer to generate a load current flowing through the load and the step of adjusting the driving signal to enable the load current to be equal to the target current so that the ultrasonic transducer is in the resonant state, until the target tissue is cut off.

13. A control device for an ultrasonic transducer, the ultrasonic transducer being connected with a load and the control device comprising:

an acquisition unit, configured to acquire a target current which flows through the load and enables the ultrasonic transducer to be in a resonant state;
a driving unit, configured to apply a driving signal to the ultrasonic transducer to generate a load current flowing through the load; and
an adjusting unit, configured to adjust the driving signal to enable the load current to be equal to the target current, so that the ultrasonic transducer is in the resonant state.

14. A surgical equipment, comprising a host and an ultrasonic transducer connected to the host, wherein the host comprises the control device according to claim 13.

15. A surgical equipment, comprising:

a processor; and
a memory, comprising one or more computer program modules, wherein
the one or more computer program modules are stored in the memory and configured to be executed by the processor, and the one or more computer program modules comprise instructions for executing the control method according to any one of claims 1 to 11.

16. A storage medium for non-temporarily storing computer-readable instructions, wherein the computer-readable instructions, when executed by a computer, are configured to realize the control method according to any one of claims 1 to 11.

Fig. 1

Fig. 2

I →

U

I2 ↓  C0

I1 ↓  L1

C1

R1

R

Fig. 3

Obtain a target current I0 which flows through a load and enables an ultrasonic transducer to be in a resonant state I0

S100

Apply a driving signal to the ultrasonic transducer to generate a load current I1 flowing through the load R

S200

Adjust the driving signal to enable the load current I1 to be equal to the target current I0, such that the ultrasonic transducer is in the resonant state

S300

Fig. 4

S300

Acquiring a driving frequency f applied to the
ultrasonic transducer f — S310

Adjusting the phase angle θ by adjusting the
driving frequency f, so that the load current I1 is
equal to the target current I0 — S320

Fig. 5

S320

Determining a target phase angle θ0 and a target
frequency f0 corresponding to the target phase
angle θ0 — S321

Adjusting the driving frequency f to enable the
adjusted driving frequency f to be equal to the
target frequency f0 and enable the adjusted phase
angle θ to be equal to the target phase angle θ0 — S322

Fig. 6

I2

I

θ (θ0)

I1(I0)

Fig. 7

S300'

S330

| Acquiring a driving current I applied to the ultrasonic transducer |
|---|

S340

| Adjusting the load current I1 by adjusting the driving current I to enable the load current I1 to be equal to the target current I0 |
|---|

Fig. 8

S3211

Acquiring a driving voltage U applied to the
ultrasonic transducer and a capacitive
reactance Xc of the ultrasonic transducer

S3212

Determining a current I2 flowing through the
static capacitor C0 according to the capacitive
reactance Xc and the driving voltage U

S3213

Determining the target phase angle θ0
according to the current I2 flowing through
the static capacitor and the target current I0

Fig. 9

S3214

Performing frequency scanning within a
preset frequency range

S3215

Acquiring a frequency that enables the
ultrasonic transducer to be in a resonant state
as the target frequency f0 during the
frequency scanning

Fig. 10

Fig. 11

## INTERNATIONAL SEARCH REPORT

| International application No. |
| --- |
| **PCT/CN2023/080376** |

### A. CLASSIFICATION OF SUBJECT MATTER

A61B17/32(2006.01)i

According to International Patent Classification (IPC) or to both national classification and IPC

### B. FIELDS SEARCHED

Minimum documentation searched (classification system followed by classification symbols)

IPC: A61B

Documentation searched other than minimum documentation to the extent that such documents are included in the fields searched

Electronic data base consulted during the international search (name of data base and, where practicable, search terms used)

CNTXT; CNKI; ENTXTC; ENTXT; VEN: 超声, 换能, 谐振, 电流, 负载, 调, 恒, 控制, 阻抗, 等于, ultrasonic, transducer, resonance, current, load, adjust+, constant

### C. DOCUMENTS CONSIDERED TO BE RELEVANT

| Category* | Citation of document, with indication, where appropriate, of the relevant passages | Relevant to claim No. |
| --- | --- | --- |
| PX | CN 114305599 A (HOCER (BEIJING) MEDICAL TECHNOLOGIES CO., LTD.) 12 April 2022 (2022-04-12) claims 1-16, description, paragraphs [0025]-[0072], and figures 1-11 | 1-16 |
| PX | CN 115005934 A (HOCER (BEIJING) MEDICAL TECHNOLOGIES CO., LTD.) 06 September 2022 (2022-09-06) claims 1-13, description, paragraphs [0038]-[0109], and figures 1-11 | 1-16 |
| X | CN 113633351 A (SHENZHEN CHENGCHUAN MEDICAL CO., LTD.) 12 November 2021 (2021-11-12) description, paragraphs [0031]-[0148], and figures 1-8 | 1, 2, 12-16 |
| A | CN 113648028 A (SHENZHEN CHENGCHUAN MEDICAL CO., LTD.) 16 November 2021 (2021-11-16) entire document | 1-16 |
| A | CN 113289880 A (TSINGHUA SHENZHEN INTERNATIONAL GRADUATE SCHOOL) 24 August 2021 (2021-08-24) entire document | 1-16 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **02 June 2023** | **16 June 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/080376**

**C.    DOCUMENTS CONSIDERED TO BE RELEVANT**

| Category* | Citation of document, with indication, where appropriate, of the relevant passages | Relevant to claim No. |
|---|---|---|
| A | US 4256987 A (TOYODA CHUO KENKYUSHO K. K.) 17 March 1981 (1981-03-17) entire document | 1-16 |
| A | JP H07185457 A (OLYMPUS OPTICAL CO.) 25 July 1995 (1995-07-25) entire document | 1-16 |

Form PCT/ISA/210 (second sheet) (July 2022)

INTERNATIONAL SEARCH REPORT
Information on patent family members

International application No.

**PCT/CN2023/080376**

| Patent document cited in search report | | | Publication date (day/month/year) | Patent family member(s) | Publication date (day/month/year) |
|---|---|---|---|---|---|
| CN | 114305599 | A | 12 April 2022 | None | |
| CN | 115005934 | A | 06 September 2022 | None | |
| CN | 113633351 | A | 12 November 2021 | None | |
| CN | 113648028 | A | 16 November 2021 | None | |
| CN | 113289880 | A | 24 August 2021 | None | |
| US | 4256987 | A | 17 March 1981 | None | |
| JP | H07185457 | A | 25 July 1995 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210249080